# EUROPEAN PATENT SPECIFICATION

(11) **EP 0 497 331 B1**
(45) Date of publication and mention of the grant of the patent: **28.08.1996**
(21) Application number: 92101501.2
(22) Date of filing: 30.01.1992
(51) Int. Cl.: A61K 47/44, A61K 9/20, A61K 9/28, A61K 31/195

(54) **Process for the preparation of tablets or film-coated tablets**
Verfahren zur Herstellung von Tabletten oder filmbeschichteten Tabletten
Procédé pour la préparation de comprimés ou de comprimés enrobés

(30) Priority: 30.01.1991 HU 31891
(43) Date of publication of application: 05.08.1992
(73) Proprietor: EGIS GYOGYSZERGYAR, H-1106 Budapest (HU)
(72) Inventor: Erdos, Sándor, Dr., H-1083 Budapest (HU); Bezzegh, Dénes, H-1143 Budapest (HU); Egri, János, H-1036 Budapest (HU); Bárczay, Erzsébet, H-1072 Budapest (HU); Magyar, Olga, H-1119 Budapest (HU); Sümeg, Katalin, H-1015 Budapest (HU)
(74) Representative: Beszédes, Stephan G., Dr.

(56) References cited:
- EP-A- 0 320 051
- US-A- 2 793 979
- US-A- 3 062 720
- US-A- 3 769 424

## Description

This invention is concerned with a process for the preparation of solid pharmaceutical compositions. More specifically, it is concerned with a process for the preparation of tablets or film-coated tablets with increased active ingredient release, said tablets comprising as active ingredients a mixture of L-3,4-di-(hydroxy)-phenylalanine {(S)-2-[amino]-3-[3',4'-di-(hydroxy)-phenyl]-propionic acid}〈levodopa〉 and L-alpha-(hydrazino)-alpha-(methyl)-3,4-di-(hydroxy)-phenyl propionic acid {(S)-2-[3',4'-di-(hydroxy)-benzyl]-2-[hydrazino]-propionic acid} 〈carbidopa〉 in a (3 to 12) : 1 weight to weight ratio.

It is known in the art that L-3,4-di-(hydroxy)-phenylalanine (levodopa), when used in combination with L-alpha-(hydrazino)-alpha-(methyl)-3,4-di-(hydroxy)-phenyl propionic acid (carbidopa), is useful in the treatment of Parkinson's disease (US-PS 3,769,424). Although in this specification there is a reference to the preparation of pharmaceutical compositions comprising as active ingredients a mixture of levodopa and carbidopa in a (0.2 to 8) : 1 weight to weight ratio, neither the composition itself nor the process for the preparation thereof have been disclosed.

In EP-A-320 051 it has been described a controlled release oral dosage formulation comprising a uniform dispersion of 5-300 mg of carbidopa, 20-1200 mg of levodopa, 0-25 mg of a tablet lubricant and optionally a pharmaceutically acceptable dye, in a polymer vehicle comprising 0-120 mg of a water soluble polymer which is hydroxypropyl cellulose Klucel LF and 0-120 mg of a less water soluble polymer which is polyvinyl acetate crotonic acid copolymer, Vinac ASB-516 with the proviso that both polymers are not 0 mg, whereby following administration the carbidopa and levodopa are released slowly and simultaneously from the formulation. Furthermore the use of higher fatty acids, such as stearic acid or palmitic acid, with the function of lubricants has been mentioned. In Example 3 microcrystalline cellulose has been used evidently as a filler but without a fatty acid.

Furthermore in US-A-2 793 979 it has been described a method of forming a sustained release pharmaceutical tablet which comprises liquefying a sustained release material comprising a solid fatty material resistant to disintegration and slowly dispersible in the gastrointestinal tract, dispersing a medicament in said liquefied material, solidifying the mass and reducing it to a powder, mixing the thus formed powder with a granulating syrup solution, granulating and drying said mixture to provide sustained release granules, mixing the thus formed sustained release granules with granules of the selected medicament free of sustained release material, all of the granules being about the same size, and tabletting the thus formed mixture thus a fatty acid, such as stearic or palmitic acid can be used as fatty time delay material. As an example of inert fillers lactose has been mentioned.

Moreover from US-A-3 062 720 it has been known a sustained release pharmaceutical tablet consisting essentially of about 1 to 70% of a water soluble medicament about 10 to 40% of a substantially water insoluble fatty material, about 5 to 70% of a filler, about 5 to 10% of a binder and about 5 to 20% of a lubricant, the tablet having a time delay number of about 250 to 350, a hardness between about 10 kg, and 3 kg, and being a substantially homogeneous tablet which retains its original shape while the soluble medicament is leached therefrom, thus leaving a network of minute channels in the tablet.

According to the requirements raised against tablets with increased active ingredient release comprising as active ingredient levodopa in combination with carbidopa, which are specified by US Pharmacopoeia XXII (page 226) being in force as from January 1, 1990, at least 80% of the active ingredient content of the tablet should be released in a 0.1 n hydrogen chloride solution within 30 minutes, under definite circumstances. The composition of the tablets complying with these requirements and the process for the preparation thereof, however, are not provided for in said pharmacopoeia.

The problem underlying to the invention was to elaborate a process for the preparation of tablets or film-coated tablets with increased active ingredient release, said tablets comprising as active ingredients a mixture of L-3,4-di-(hydroxy)-phenylalanine and L-alpha-(hydrazino)-alpha-(methyl)-3,4-di-(hydroxy)-phenyl propionic acid in a (3 to 12) : 1 weight to weight ratio, ensuring a release of 80% by weight of the active ingredient content of the tablets, under the circumstances specified by US Pharmacopoeia XXII (page 226), within 5 to 10 minutes in a 0.1 n hydrogen chloride solution, and furthermore meeting the usual requirements of quality, that is, particularly being perfect and undamaged in appearance and properly solid.

Surprisingly this has been attained by the invention.

The subject matter of the invention is a process for the preparation of tablets or film-coated tablets with increased active ingredient release, said tablets comprising as active ingredients a mixture of L-3,4-di-(hydroxy)-phenylalanine and L-alpha-(hydrazino)-alpha-(methyl)-3,4-di-(hydroxy)-phenyl propionic acid in a (3 to 12) : 1 weight to weight ratio using hydrophilic agents and optionally auxiliary agents, drying and granulating the obtained mixture and compressing it into tablets, characterized by admixing any of L-3,4-di-(hydroxy)-phenylalanine and L-alpha-(hydrazino)-alpha-(methyl)-3,4-di-(hydroxy)-phenyl propionic acid, or both of them, with 1 or both of the hydrophilic diluents lactose and/or microcrystalline cellulose, and optionally with 1 or more colouring agent(s), blending the mixture thus obtained with 1 or more auxiliary agent(s) conventionally used for the preparation of tablets and optionally with the other active ingredient, moreover with a solution of stearin in an organic solvent, optionally containing 1 or more auxiliary agent(s) conventionally used for the preparation of tablets, drying and granulating the mixture thus obtained, optionally admixing 1 or more further auxiliary agent(s) to the dry granulate, compressing it into tablets comprising 50 to 70% by weight of active ingredient and, if desired, applying a film-coating onto the surface of the tablets thus obtained.

The term "L-alpha-(hydrazino)-alpha-(methyl)-3,4-di-(hydroxy)-phenyl propionic acid" encompasses both its anhydrous form and its monohydrate. The use of the latter is preferred.

A fundamental difference between the compositions of the Prior Art and the compositions according to the invention is that the former are sustained released ones, whereas the latter are such with quick release of the active principles. Thus as far as microcrystalline cellulose has been mentioned in the Prior Art it has not been used for securing a quick release of the active principle. Furthermore a combined use of a hydrophilic diluent has not been indicated in the Prior Art. An additional difference to EP-A-320 051 is the use of lactose and/or microcrystalline cellulose as diluent(s) not described in the former. A further difference to US-A-2 793 979 and US-A-3 062 720 is that in the latter not the active principles of the compositions prepared according to the invention have been used for which, however, the compositions prepared according to the invention are specific.

Advantageously a concentration of the stearin in the alkanol of 10 to 15% by weight is used.

Preferably an alkanol comprising 2 to 4 carbon atoms is used as organic solvent.

Suitably the mixture of L-3,4-di-(hydroxy)-phenylalanine and/or L-alpha-(hydrazino)-alpha-(methyl)-3,4-di-(hydroxy)-phenyl propionic acid and of hydrophilic agent(s) and optionally colouring agent(s) is dried before further processing.

In the process according to the invention the rapid release of the active ingredients is ensured mainly by the admixing of any of the active ingredients, or both of them, with the hydrophilic diluent(s). Advantageously this is carried out by exerting a shear force, that is, by passing the mixture through e.g. a hammer mill or a cogged plate grinder, or by using blade mixers or high-speed kneading machines.

The suitable appearance and mechanical stability of the tablets according to the invention is ensured mainly by carrying out the aggregation with stearin dissolved in an organic solvent, wherein the stearin is used preferably in an amount of 1.5 to 2.5% by weight related to the mass of the ready-made tablet. As organic solvent particularly preferably ethanol and/or isopropanol is/are used. The organic solution of stearin may contain a binder, such as methylmethacrylic acid ester polymer, too. This step attributes to the rapid release of the active ingredients.

The above specified effects of the application of stearin could not be aforeseen, as according to the own experiences directed to the preparation of tablets with increased active ingredient release comprising as active ingredient a mixture of L-3,4-di-(hydroxy)-phenylalanine and L-alpha-(hydrazino)-alpha-(methyl)-3,4-di-(hydroxy)-phenyl propionic acid in a (3 to 12) : 1 weight to weight ratio without applying stearin dissolved in an organic solvent for the aggregation, the tablets adhere to the punch faces during compression. So in this way neither tablets of appropriate appearance can be obtained nor the release of the active ingredients achieves the desired rate.

Stearin is used as lubricant for the preparation of tablets (J. Am. Pharm. Ass., 45 (1), 51 [1956]; 49 (1), 35 [1960]) in an amount of about 1% by weight, preferably in an amount not exceeding 1% by weight. According to practical experiences the mere presence of stearin may lead to the sticking of tablets, that is why it is surprising that by the application thereof this phenomenon can be eliminated. Besides, as stearin is insoluble in water, it could have been expected that in the presence thereof the release of the active ingredients would be retarded.

According to the invention stearin is used suitably in an amount of at least 1.5% by weight related to the weight of the ready-made tablet.

As conventional pharmaceutical auxiliary agent(s) after the treatment with the hydrophilic diluent(s) e.g. [a] further diluent(s), cellulose, [a] disintegrating agent(s), preferably carboxymethylcellulose and/or [a] low-substituted hydroxypropylcellulose(s), and/or [a] binder(s), preferably [a] methylmethacrylic acid ester polymer(s) and/or methylmethacrylic acid polymer, is/are used.

It is considered preferable to apply a low-substituted hydroxypropylcellulose, which may act both as a binder and as disintegrant.

If both of the active ingredients are treated with the hydrophilic solvent(s), only the above specified auxiliary agent(s) conventionally used for the preparation of tablets and the stearin solution are added to them. If only one of the active ingredients, generally L-alpha-(hydrazino)-alpha-(methyl)-3,4-di-(hydroxy)-phenyl propionic acid, is treated with the hydrophilic diluent(s), the other ingredient, furthermore the auxiliary agent(s) and the stearin solution are introduced to the mixture thus obtained.

After granulation as [a] further auxiliary agent(s) conventionally used for the preparation of tablets e.g. [a] disintegrant(s), preferably carboxymethylcellulose, antiadhesive(s), preferably talc, glidant(s), preferably colloidal silicon dioxide, and/or lubricant(s), preferably magnesium stearate, can be used.

The optional film-coating of the tablets obtained during the tableting procedure can be carried out by methods known per se.

When tablets coloured in their material are to be produced, it is preferable to blend the colouring agent(s) with the active ingredient(s) and with the hydrophilic diluent(s) by exerting a shear force. One may also proceed by colouring first the hydrophilic diluent(s) with the aqueous solution of [a] colouring agent(s), then after drying blending the mixture thus obtained with the active ingredient(s) by exerting a shear force. In both cases the procedure is continued by adding further auxiliary agent(s) to the mixture and kneading it with the stearin solution.

Colouring may also be ensured by film-coating.

The process according to the invention provides tablets with rapid active ingredient release. The appropriate quality of the tablets is ensured without an unjustified increase of the amount of the auxiliary agents, so the active ingredient content of the tablets according to the invention may be as high as 70%.

The invention is illustrated in detail by the following Examples.

### Example 1

A mixture of 1720 g of lactose and 1720 g of porous microcrystalline cellulose is thoroughly moistened with an aqueous solution of 2 g of indigo carmine, then dried. The coloured mixture thus obtained is admixed with 10 kg of L-3,4-di-(hydroxy)-phenylalanine and 1080 g of L-alpha-(hydrazino)-alpha-(methyl)-3,4-di-(hydroxy)-phenyl propionic acid monohydrate, then passed through a cogged plate grinder of Alpin type. 800 g of methylmethacrylic acid ester polymer (Eudragit® L 100) and 160 g of cross-linked carboxymethylcellulose are added to the crushed product thus obtained and it is kneaded in a kneading machine with a solution of 280 g of stearin in 2200 g of ethanol. Then it is dried and regranulated. 400 g of talc, 80 g of magnesium stearate, 20 g of colloidal silicon dioxide (Aerosil®, produced by Degussa) and 140 g of cross-linked carboxymethylcellulose are admixed to the dry granulate and the mixture is compressed into tablets of 10 mm diameter. Thus, tablets weighing 0.41005 g and having the following composition are obtained:

| | |
|---|---|
| L-3,4-di-(hydroxy)-phenylalanine | 0.250 g |
| L-alpha-(hydrazino)-alpha-(methyl)-3,4-di-(hydroxy)-phenyl propionic acid monohydrate | 0.027 g |
| lactose | 0.043 g |
| microcrystalline cellulose | 0.043 g |
| colouring agent (indigo carmine) | 0.00005 g |
| methylmethacrylic acid ester polymer | 0.020 g |
| cross-linked carboxymethylcellulose | 0.0075 g |
| stearin | 0.007 g |
| talc | 0.010 g |
| magnesium stearate | 0.002 g |
| colloidal silicon dioxide | 0.0005 g |
| | $\overline{\text{0.41005 g}}$ |

The active ingredient release of the tablets thus obtained was examined by the method specified in US Pharmacopoeia XXII (page 226). 85% by weight of the active ingredients were released within 5 minutes.

### Example 2

A mixture of 1204 g of lactose and 3393.6 g of microcrystalline cellulose is thoroughly moistened with an aqueous solution of 8.4 g of indigo carmine and dried. The coloured mixture thus obtained is admixed with 7 kg of L-3,4-di-(hydroxy)-phenylalanine and 756 g of L-alpha-(hydrazino)-alpha-(methyl)-3,4-di-(hydroxy)-phenyl propionic acid monohydrate, then passed through a cogged plate grinder of Alpin type. To the crushed mixture 560 g of methylmethacrylic acid ester polymer (Eudragit® L 100) and 434 g of low-substituted hydroxypropylcellulose are added, the mixture thus obtained is kneaded in a kneading machine with a solution of 224 g of stearin in 2200 g of ethanol, dried and granulated. To the dry granulate 336 g of talc, 70 g of magnesium stearate and 14 g of colloidal silicon dioxide are added and the mixture is compressed into tablets of 8 mm diameter. Thus, tablets weighing 0.194 g and having the following composition are obtained:

| | |
|---|---|
| L-3,4-di-(hydroxy)-phenylalanine | 0.1000 g |
| L-alpha-(hydrazino)-alpha-(methyl)-3,4-di-(hydroxy)-phenyl propionic acid monohydrate | 0.0108 g |
| lactose | 0.0172 g |
| microcrystalline cellulose | 0.04848 g |
| colouring agent (indigo carmine) | 0.00012 g |
| methylmethacrylic acid ester polymer | 0.0080 g |
| low-substituted hydroxypropylcellulose | 0.0002 g |
| stearin | 0.0032 g |
| talc | 0.0048 g |
| magnesium stearate | 0.0010 g |
| colloidal silicon dioxide | 0.0002 g |
| | $\overline{\text{0. 1940 g}}$ |

The active ingredient release of the tablets thus obtained was examined by the method specified in US Pharmacopoeia XXII (page 226). 83% by weight of the active ingredient were released within 5 minutes.

### Example 3

Tablets having the following composition are prepared by the method specified in Example 2:

| | |
|---|---|
| L-3,4-di-(hydroxy)-phenylalanine | 0.1000 g |
| L-alpha-(hydrazino)-alpha-(methyl)-3,4-di-(hydroxy)-phenyl propionic acid monohydrate | 0.0270 g |
| lactose | 0.0200 g |
| microcrystalline cellulose | 0.0520 g |
| colouring agent (quinoline yellow) | 0.0008 g |
| methylmethacrylic acid ester polymer | 0.0100 g |
| low-substituted hydroxypropylcellulose | 0.0060 g |
| stearin | 0.0040 g |
| talc | 0.0040 g |
| magnesium stearate | 0.0010 g |
| colloidal silicon dioxide | 0.0002 g |
| | $\overline{\text{0.2250 g}}$ |

### Example 4

A mixture of 108 g of L-alpha-(hydrazino)-alpha-(methyl)-3,4-di-(hydroxy)-phenyl propionic acid monohydrate and 172 g of lactose is passed through a cogged plate grinder of Alpin type, then it is blended with 1000 g of L-3,4-di-(hydroxy)-phenylalanine, 166 g of microcrystalline cellulose, 48 g methylmethacrylic acid ester polymer (Eudragit® L 100) and 16 g of cross-linked carboxymethylcellulose in a kneading machine. The mixture is moistened with a solution of 28 g of stearin in 220 g of ethanol, kneaded thoroughly, dried and regranulated. To the dry granulate 52 g of cross-linked carboxymethylcellulose, 40 g of talc, 8 g of colloidal silicon dioxide (Aerosil®, produced by Degussa) and 8 g of magnesium stearate are added and the mixture is compressed into tablets of 10 mm diameter. Thus, tablets weighing 0.410 g are obtained with the following composition:

| | |
|---|---|
| L-3,4-di-(hydroxy)-phenylalanine | 0.250 g |
| L-alpha-(hydrazino)-alpha-(methyl)-3,4-di-(hydroxy)-phenyl propionic acid monohydrate | 0.0270 g |
| lactose | 0.043 g |
| microcrystalline cellulose | 0.040 g |
| methylmethacrylic acid ester polymer | 0.012 g |
| cross-linked carboxymethylcellulose | 0.017 g |
| stearin | 0.007 g |
| talc | 0.010 g |
| colloidal silicon dioxide | 0.002 g |
| magnesium stearate | 0.002 g |
| | 0.410 g |

The active ingredient release of the tablets thus obtained was examined by the method specified in US Pharmacopoeia XXII (page 226). 80% by weight of the active ingredient were released within 5 minutes.

### Example 5

One proceeds according to Example 4 except that 24 g of methylmethacrylic acid polymer (Eudragit® L 100) are used, 28 g of stearin are dissolved in 192 g of a 12.5% by weight solution of methylmethacrylic acid ester polymer in isopropanol (Eudragit® L 12.5 P) and moistening is carried out with the solution obtained.

### Example 6

A mixture of 400 g of L-3,4-di-(hydroxy)-phenylalanine, 108 g of L-alpha-(hydrazino)-alpha-(methyl)-3,4-di-(hydroxy)-phenyl propionic acid monohydrate and 104 g of lactose is blended in a hammer mill. Then it is kneaded in a kneading machine with 96 g of microcrystalline cellulose, 32 g of methylmethacrylic acid ester polymer (Eudragit® L 100), 4 g of cross-linked carboxymethylcellulose and a solution of 16 g of stearin in 125 g of ethanol, dried and granulated. To the dry granulate 48 g of cross-linked carboxymethylcellulose, 24 g of talc, 4 g of colloidal silicon dioxide and 4 g of magnesium stearate are admixed and the mixture is compressed into tablets having 8 mm diameter. Thus, tablets weighing 0.21 g are obtained with the following composition:

| | |
|---|---|
| L-3,4-di-(hydroxy)-phenylalanine | 0.100 g |
| L-alpha-(hydrazino)-alpha-(methyl)-3,4-di-(hydroxy)-phenyl propionic acid monohydrate | 0.027 g |
| lactose | 0.026 g |
| microcrystalline cellulose | 0.024 g |
| methylmethacrylic acid ester polymer | 0.008 g |
| cross-linked carboxymethylcellulose | 0.013 g |
| stearin | 0.004 g |
| talc | 0.006 g |
| colloidal silicon dioxide | 0.001 g |
| magnesium stearate | 0.001 g |
| | $\overline{\text{1.210 g}}$ |

The active ingredient release of the tablets thus obtained was examined by the method specified in US Pharmacopoeia XXII (page 226). 80% by weight of the active ingredient were released within 5 minutes.

## Claims

1. A process for the preparation of tablets or film-coated tablets with increased active ingredient release, said tablets comprising as active ingredients a mixture of L-3,4-di-(hydroxy)-phenylalanine and L-alpha-(hydrazino)-alpha-(methyl)-3,4-di-(hydroxy)-phenyl propionic acid in a (3 to 12) : 1 weight to weight ratio using hydrophilic agents and optionally auxiliary agents, drying and granulating the obtained mixture and compressing it into tablets, characterized by admixing any of L-3,4-di-(hydroxy)-phenylalanine and L-alpha-(hydrazino)-alpha-(methyl)-3,4-di-(hydroxy)-phenyl propionic acid, or both of them, with 1 or both of the hydrophilic diluents lactose and/or microcrystalline cellulose, and optionally with 1 or more colouring agent(s), blending the mixture thus obtained with 1 or more auxiliary agent(s) conventionally used for the preparation of tablets and optionally with the other active ingredient, moreover with a solution of stearin in an organic solvent, optionally containing 1 or more auxiliary agent(s) conventionally used for the preparation of tablets, drying and granulating the mixture thus obtained, optionally admixing 1 or more further auxiliary agent(s) to the dry granulate, compressing it into tablets comprising 50 to 70% by weight of active ingredient and, if desired, applying a film-coating onto the surface of the tablets thus obtained.

2. A process as claimed in claim 1, characterized by using an alkanol comprising 2 to 4 carbon atoms as organic solvent.

## Patentansprüche

1. Verfahren zur Herstellung von Tabletten oder filmbeschichteten Tabletten mit erhöhter Wirkstoffbestandteil-Abgabe, wobei bei den Tabletten, die als Wirkstoffbestandteile eine Mischung aus L-3,4-Di-(hydroxy)-phenylalanin und L-α-(Hydrazino)-α-(methyl)-3,4-di-(hydroxy)-phenylpropionsäure in einen Gewichtsverhältnis (w/w) von (3 - 12) : 1 enthalten, hydrophile Mittel und gegebenenfalls Hilfsmittel, Trocknen und Granulieren der erhaltenen Mischung und Pressen dieser zu Tabletten zur Anwendung kommen, **gekennzeichnet durch** Vermischen entweder von L-3,4-Di-(hydroxy)-phenylalanin oder von L-α-(Hydrazino)-α-(methyl)-3,4-di-(hydroxy)-phenylpropionsäure, oder von beiden, mit 1 oder beiden der hydrophilen Verdünnungsmittel Lactose und/oder mikrokristalliner Cellulose und gegebenenfalls mit 1 oder mehreren Färbemitteln, Vermischen der derart erhaltenen Mischung mit 1 oder mehreren herkömmlicherweise für die Herstellung von Tabletten verwendeten Hilfsmitteln und gegebenenfalls mit dem anderen Wirkstoffbestandteil, außerdem mit einer Lösung von Stearin in einem organischen Lösemittel, gegebenenfalls 1 oder mehrere herkömmlicherweise für die Herstellung von Tabletten verwendete Hilfsstoffe enthaltend, Trocknen und Granulieren der derart erhaltenen Mischung, gegebenenfalls Einmischen von 1 oder mehreren weiteren Hilfsstoffen zum trockenen Granulat, Pressen desselben zu Tabletten, die 50 bis 70 Gew.-% Wirkstoffbestandteil enthalten, und, falls gewünscht, Auftragen einer Filmbeschichtung auf der Oberfläche der derart erhaltenen Tabletten.

2. Verfahren nach Anspruch 1, gekennzeichnet durch die Verwendung eines Alkanols mit 2 bis 4 Kohlenstoffatomen als organisches Lösemittel.

## Revendications

1. Un procédé de préparation de comprimés ou comprimés revêtus par un film à libération accélérée de l'ingrédient actif, ces comprimés contenant à titre d'ingrédients actifs un mélange de L-3,4-di-(hydroxy)-phénylalanine et d'acide L-alpha-(hydrazino)-alpha-(méthyl)-3,4-di-(hydroxy)-phényl propionique utilisés en un rapport pondéral de 3/1 à 12/1 associé à des agents hydrophiles et éventuellement à des adjuvants, le mélange étant séché, broyé puis compressé pour former des comprimés, ce procédé étant caractérisé en ce qu'il comprend le mélange de la L-3,4-di-(hydroxy)-phénylalanine ou de l'acide L-alpha-(hydrazino)-alpha-(méthyl)-3,4-di-(hydroxy)-phényl propionique ou de ces deux composés avec les diluants hydrophiles lactose et ou cellulose microcristalline, et éventuellement avec un ou plusieurs colorants, le mélange du mélange ainsi obtenu avec un ou plusieurs adjuvants classiquement utilisés pour la préparation de comprimés et éventuellement avec l'autre ingrédient actif, et en outre avec une solution de stéarine dans un solvant organique contenant éventuellement un ou plusieurs adjuvants classiquement utilisés pour la préparation de comprimés, le séchage et le broyage du mélange ainsi obtenu, éventuellement l'addition de un ou plusieurs adjuvants au granulat sec, la compression du mélange résultant en comprimés comprenant 50 à 70% en poids d'ingrédient actif et, le cas échéant, l'application d'un film de revêtement sur la surface des comprimés ainsi obtenus.

2. Un procédé ainsi que revendiqué dans la revendication 1, caractérisé en ce que l'on utilise comme solvant organique un alcanol comprenant 2 à 4 atomes de carbone.
